**Europäisches Patentamt**

(19) **European Patent Office**

**Office européen des brevets**

(11) Veröffentlichungsnummer: **0 011 180**
**B1**

(12) # EUROPÄISCHE PATENTSCHRIFT

(45) Veröffentlichungstag der Patentschrift:
13.04.83

(51) Int. Cl.³: **A 61 B 5/14**

(21) Anmeldenummer: **79104216.1**

(22) Anmeldetag: **30.10.79**

(54) **Blutaufnahmevorrichtung.**

(30) Priorität: **09.11.78 DE 2848535**
**17.02.79 DE 2906209**

(43) Veröffentlichungstag der Anmeldung:
**28.05.80 Patentblatt 80/11**

(45) Bekanntmachung des Hinweises auf die Patenterteilung:
**13.04.83 Patentblatt 83/15**

(84) Benannte Vertragsstaaten:
**AT BE CH FR GB IT LU NL SE**

(56) Entgegenhaltungen:
**DE-A-2 558 311**
**DE-B-1 297 361**
**DE-B-2 439 218**
**FR-A-1 317 488**
**US-A-2 737 812**
**US-A-2 965 255**
**US-A-3 525 264**
**US-A-3 926 521**
**US-A-4 091 802**

(73) Patentinhaber: **Walter Sarstedt**
**Kunststoff-Spritzgusswerk,**
**D-5223 Nümbrecht/Rommelsdorf (DE)**

(72) Erfinder: **Sarstedt, Walter,**
**D-5223 Nümbrecht/Rommelsdorf (DE)**

(74) Vertreter: **Dipl.-Phys.Dr. Manitz Dipl.-Ing. Finsterwald**
**Dipl.-Ing. Grämkow Dipl.-Chem.Dr. Heyn**
**Dipl.-Phys.Rotermund Morgan B.Sc.(Phys.),**
**Robert-Koch-Strasse 1, D-8000 München 22 (DE)**

BUNDESDRUCKEREI BERLIN

Blutaufnahmevorrichtung

Die Erfindung betrifft eine Blutaufnahmevorrichtung, bestehend aus einer beiderseits offenen Kapillare festgelegten Volumens, welche wenigstens am vorderen Ende derart eng ausgebildet ist, daß sie das aufzunehmende Blut durch Kapillarwirkung selbsttätig in ihren Innenraum einsaugt, sobald das vordere Ende mit der Blutentnahmestelle in Berührung gebracht wird und die nach hinten einstückig derart in einen Halter übergeht, daß das vordere Ende der Kapillare über den Halter axial hinausragt.

Es ist bereits eine Blutaufnahmevorrichtung dieser Art bekannt (US-A-2 737 812), bei der an ein kapillarförmiges Mundstück über eine konische Erweiterung ein größerer Aufnahmeraum anschließt. Beim Entnehmen von Blut dient das erweiterte Ende gleichzeitig als Halter für das kapillarförmige Mundstück.

Nachteilig bei den bekannten, mit Kapillarwirkung arbeitenden Blutaufnahmevorrichtungen ist, daß am hinteren Ende der Kapillare nach dem Füllen mit Blut eine mehr oder weniger überstehende Blutkuppe entsteht, wobei das Volumen der Kuppe von der Zusammensetzung und der Temperatur des Blutes sowie anderen Einflüssen abhängt. Insbesondere bei kleinen Volumina der Kapillare können diese Volumenschwankungen prozentual so groß werden, daß die hierdurch entstehenden Dosierfehler nicht mehr in Kauf genommen werden können.

Das Ziel der vorliegenden Erfindung besteht somit darin, eine Blutaufnahmevorrichtung der eingangs genannten Gattung zu schaffen, mit der unabhängig von Schwankungen der Zusammensetzung und der Temperatur des Blutes sowie anderer Einflußfaktoren eine genaue Dosierung der aufgenommenen Blutmenge gewährleistet ist.

Zur Lösung dieser Aufgabe sieht die Erfindung vor, daß ein Verschlußstift in dem vom Mantel der Kapillare ausgehenden, Spülfenster aufweisenden Halter auf dem hinteren Ende der Kapillare unter Freilassung kleiner Luftaustrittsöffnungen abnehmbar angeordnet ist oder daß das hintere Ende der Kapillare durch eine Querwand verschlossen ist, in der sich eine oder mehrere kleine Luftaustrittsöffnungen befinden.

Aufgrund dieser Ausbildung wird wegen der kleinen Luftaustrittsöffnungen das Füllen der Kapillare mit Blut nicht behindert. Andererseits sind die sich an den Luftaustrittsöffnungen noch bildenden Blutkuppen nach der Füllung so klein, daß sie keine merklichen Dosierfehler hervorrufen. Es wird somit ohne eine Behinderung des Blutaufnahmevorganges eine wesentlich genauere Dosierung gewährleistet.

Besonders bevorzugt ist es, wenn die Kapillare sich nach hinten konisch erweitert, jedoch nur so weit, daß das eingesaugte Blut in waagerechter Lage der Kapillare nicht von selbst auslöst. Auf diese Weise kann die Kapillare bei unveränderter Länge eine größere Blutmenge aufnehmen.

Bei dieser Ausführungsform ist der Verschlußstift bzw. die Querwand am hinteren Ende besonders wichtig, weil durch die Erweiterung die überstehende Blutkuppe noch größer als bei einer normalen Kapillare ist.

Da nach der Blutabnahme das entnommene Blut aus der erfindungsgemäßen Blutaufnahmevorrichtung in einem Probenröhrchen ausgespült werden soll, ist zweckmäßig am hinteren Ende des Verschlußstiftes einstückig ein axial verlaufender Führungs- und Haltestab angeordnet. Dieser kann nach dem Einbringen in das mit einer Verdünnungsflüssigkeit gefüllte Probenröhrchen etwas nach hinten gezogen werden, wodurch das Verschlußstück von dem hinteren Ende der Kapillare abgehoben wird und so das Blut ausgespült werden kann. Hierbei wird auch das am Verschlußstück haftende Blut mit abgespült. Damit der Verschlußstift in jeder gewünschten Position innerhalb des Halters festgelegt werden kann, ist nach einer weiteren vorteilhaften Ausführungsform vorgesehen, daß der Haltestab klemmend, aber axial verschiebbar im Halter angeordnet ist. Dabei ist es zweckmäßig, eine Rastung vorzusehen, die ein Zurückziehen des Haltestabes um ein bestimmtes, festgelegtes Maß ermöglicht.

Der Haltestab ist vorzugsweise als verlängerter Handgriff ausgebildet und gestattet dann beim Ausspülen der entnommenen Blutprobe ein bequemes Halten und Bewegen der Kapillare in dem Spülgefäß.

Vorzugsweise ist der Haltestab an seinem unteren Ende mit mindestens drei, etwa radial nach außen vorstehenden Führungsflügeln versehen, die das untere Ende des Haltestabes im Halter führen.

Vorzugsweise wird weiterhin vorgeschlagen, daß beide Enden der Kapillare freistehen. Dadurch werden Benetzungen der die Kapillarenden umgebenden Teile mit Blut und damit größere Dosierfehler vermieden. Außerdem kann das vordere Ende der Kapillare, wenn es freisteht, also axial über den Halter hinausragt, nach der Blutentnahme vorsichtig abgewischt werden. Die Verschmutzung und die sich daraus ergebenden Dosierfehler werden außerdem dadurch geringgehalten, daß man das vordere Ende der Kapillare außen mit einem verhältnismäßig steilen Konus versieht, der eine starke Verringerung der Wandstärke der Kapillare in der Nähe der Öffnung zur Folge hat.

Kapillare und Halter sind vorzugsweise aus Kunststoff, insbesondere aus transparentem Kunststoff, hergestellt, wobei selbstverständlich auf eine gute Benetzbarkeit des Werkstoffes zu achten ist.

Der Halter weist zweckmäßig zwei einander diametral gegenüberliegende und parallel zur Achse der Kapillare oder etwa parallel dazu verlaufende Stege auf, die zwischen sich zwei größere Fenster freilassen und vorzugsweise oberseitig in ein geschlossenes Rohr übergehen, das zur Aufnahme des Abschlußstiftes bzw. seines Haltestabes dient und andererseits eine bequeme Handhabung ermöglicht.

Der Abschlußstift liegt entweder glatt auf der hinteren Öffnung der Kapillare auf, wobei er selbstverständlich an seinem Umfang Öffnungen freiläßt, oder er hat einen Zentriervorsprung, der entweder kalottenförmig oder pyramidenförmig ausgebildet ist und in die Öffnung der Kapillare hineinragt.

Vorzugsweise ist bei einer pyramidenförmigen Ausbildung des Zentriervorsprunges vorgesehen, daß von den Dachkanten der Pyramide Anschlagnasen mit radial verlaufenden Anschlagflächen vorstehen. Diese Anschlagnasen halten dann den in den Halter eingeschobenen Abschlußstift mit seiner Pyramidenfläche in einem vorbestimmten Abstand von der hinteren Öffnung der Kapillare.

Sofern die erfindungsgemäße Blutaufnahmevorrichtung so ausgebildet ist, daß das hintere Ende der Kapillare durch eine Querwand verschlossen ist, in der sich eine oder mehrere kleine Luftaustrittsöffnungen befinden, kann die Querwand entweder konvex, wobei sich eine Luftaustrittsöffnung in ihrer Mitte befindet, oder konkav ausgebildet sein, wobei sich eine oder mehrere Luftaustrittsöffnungen an ihrem Umfang befinden. Die Flüssigkeitskuppen, die sich an diesen kleinen Luftaustrittsöffnungen bilden, haben ein derart kleines Volumen gegenüber dem Gesamtvolumen der Kapillare, daß praktisch keine Dosierfehler mehr entstehen.

Die Erfindung wird nachstehend in Ausführungsbeispielen anhand der Zeichnung näher erläutert. Dabei zeigt

Fig. 1 einen Längsschnitt durch eine erfindungsgemäße Kapillare mit Halter und Abschlußstift,

Fig. 2 eine vergrößerte Teildarstellung der Kapillare mit anschließenden Stegen, jedoch ohne Abschlußstift,

Fig. 3 bis 5 dieselbe Teildarstellung, jedoch mit aufgesetztem Abschlußstift mit pyramidenförmigem Zentriervorsprung, wobei Fig. 3 einen Schnitt längs der Linie III-III in Fig. 4 ist, während Fig. 4 einen Schnitt längs der Linie IV-IV in Fig. 3 und Fig. 5 ein Schnitt längs der Linie V-V in Fig. 3 ist,

Fig. 6 einen Teilschnitt ähnlich Fig. 2 und Fig. 4 mit eingesetztem Abschlußstift, der hier jedoch einen kugelkalottenförmigen Zentriervorsprung besitzt,

Fig. 7 einen Schnitt längs der Linie VII-VII in Fig. 6,

Fig. 8 einen Teilschnitt durch das hintere Ende der Kapillare mit aufgesetztem Abschlußstift ohne Zentriervorsprung,

Fig. 9 einen Schnitt längs der Linie IX-IX in Fig. 8,

Fig. 10 eine Teildarstellung des unteren bzw. vorderen Endes eines Abschlußstiftes mit Mulde,

Fig. 11 eine Ansicht in Richtung des Pfeiles XI in Fig. 10,

Fig. 12 eine teilweise aufgebrochene Seitenansicht eines mit Stopfen verschlossenen Proberöhrchens mit einem in den Stopfen eingesteckten Halter mit Kapillare,

Fig. 13 eine Seitenansicht einer anderen Ausführungsform der Kapillare mit Halter und Abschlußstift,

Fig. 14 eine vergrößerte Teilansicht auf den mit Führungsflügeln versehenen unteren Teil des Abschlußstiftes,

Fig. 15 eine Unteransicht auf den Unterteil des Abschlußstiftes, gesehen in Richtung des Pfeiles XV in Fig. 14,

Fig. 16 einen Teilschnitt durch eine andere Ausführungsform der Kapillare mit Halter,

Fig. 16a und Fig. 16b Teildarstellungen mit einer abgewandelten Ausführung der hinteren Querwand der Kapillare,

Fig. 17 einen Teilschnitt durch eine Ausführungsform ähnlich Fig. 16 mit einer andersartigen Luftaustrittsöffnung,

Fig. 17a eine abgewandelte Ausführung der hinteren Querwand,

Fig. 18 und 18a Teildarstellungen weiterer Ausführungsformen der Kapillare mit Querwand und

Fig. 18b eine Draufsicht auf die Querwand der Ausführung nach Fig. 18a.

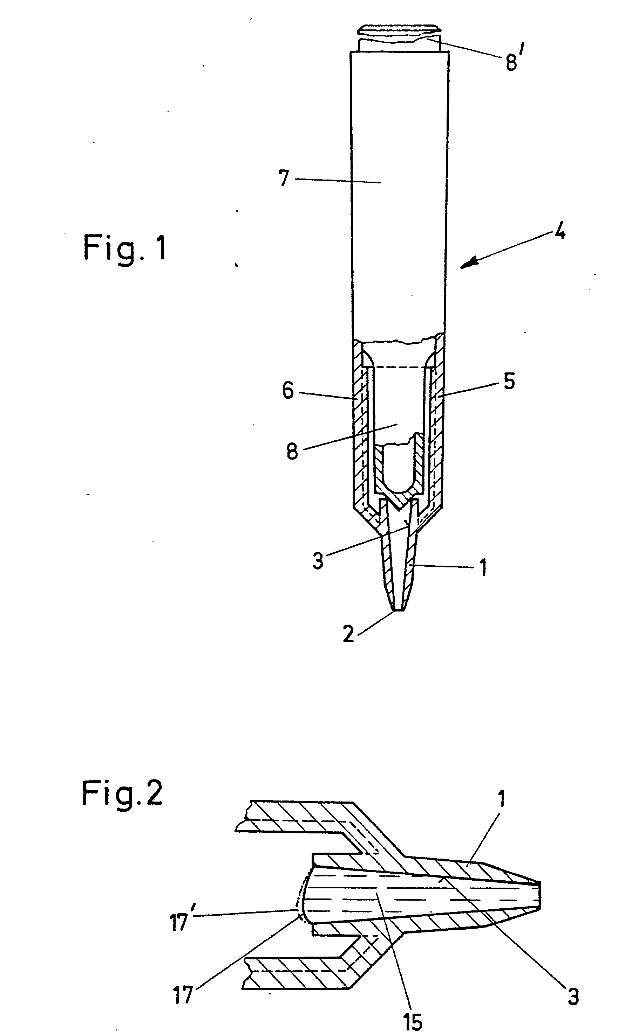

Die in den Fig. 1 bis 5 dargestellte Vorrichtung umfaßt eine Kapillare 1, deren Öffnung 3 konisch zum äußeren Ende 2 hin ausläuft, und einen mit dieser einstückig verbundenen Halter 4, beide aus transparentem Kunststoff hergestellt. Der Halter 4 weist zwei einander diametral gegenüberliegende Stege 5 und 6 auf, die an ihren in Fig. 1 oberen Enden in ein Rohr 7 übergehen.

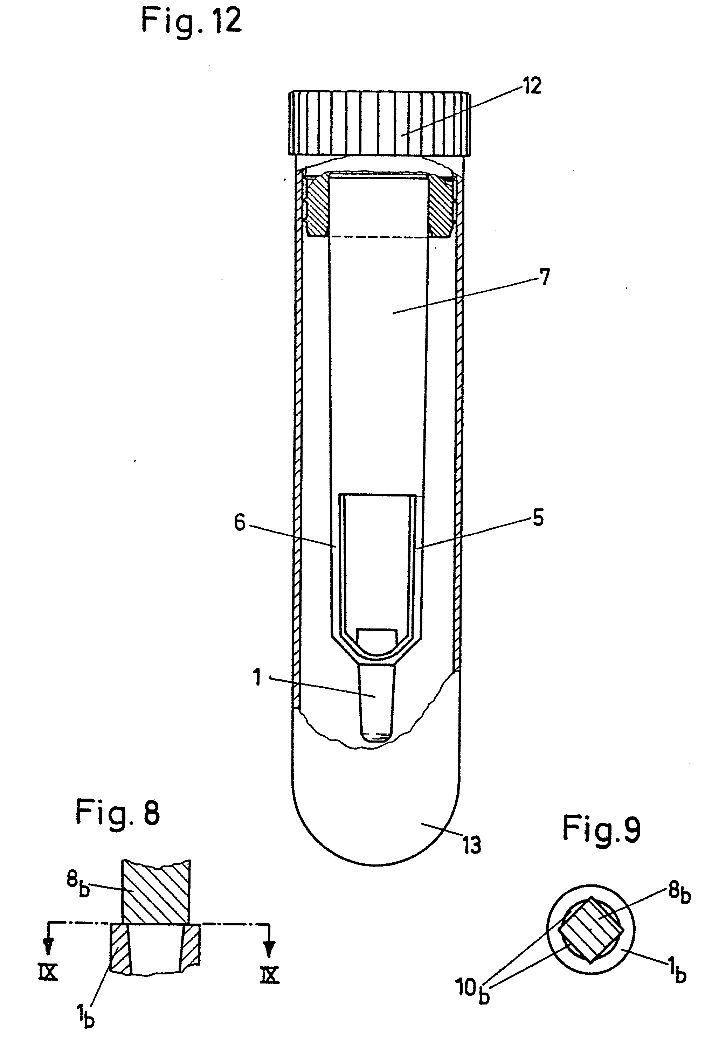

Die obere bzw. hintere Öffnung der Kapillare 1 wird durch einen Verschluß- oder Abschlußstift 8 fast vollständig verschlossen, wobei jedoch Luftaustrittsöffnungen freibleiben. Dieser Abschlußstift geht an seinem hinteren bzw. in Fig. 1 oberen Ende einstückig in einen Haltestab 8' über, der in Fig. 1 abgebrochen dargestellt ist, in Wirklichkeit aber das Rohr 7 mehr oder weniger überragt. In einer speziellen Ausführungsform ragt dieser Haltestab 8' so weit über die Oberkante des Rohres 7 hinaus, daß man ihn als Handgriff während des Umrührens und Ausspülens der Kapillare in dem in Fig. 12 dargestellten Proberöhrchen, das weiter unten noch näher erläutert wird, verwenden kann.

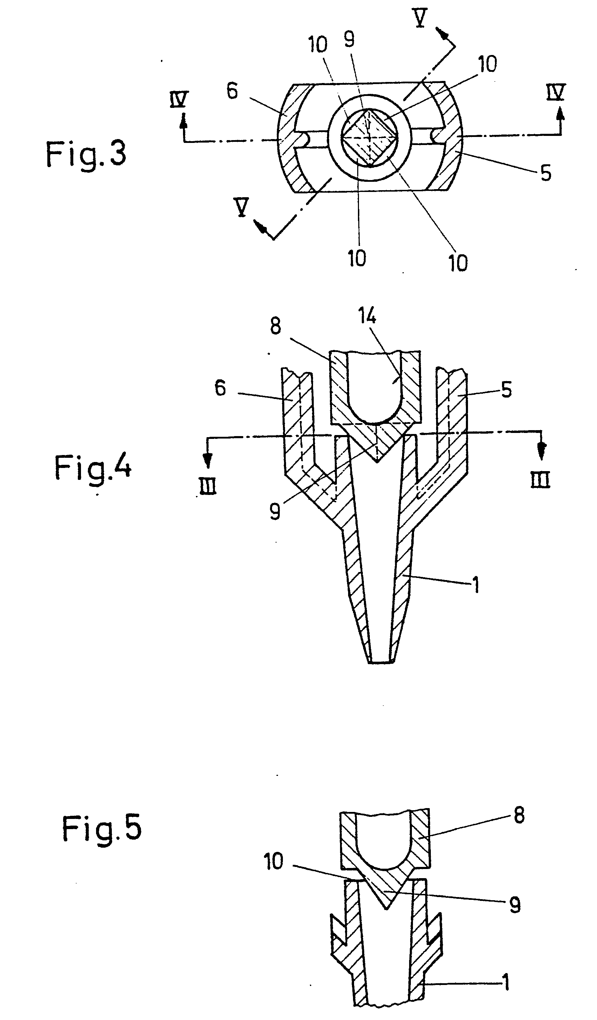

Während der Blutentnahme wird die Vorrichtung so gehalten, daß das Ende der Kapillare 1 schräg abwärts zeigt oder noch besser etwa mit seiner Achse horizontal eingestellt ist. Sofern dann kein Abschlußstift 8, wie in Fig. 1 dargestellt, eingesetzt wäre und die Blutprobe 15 durch Kapillarwirkung am vorderen Ende der Kapillare 1 eingesaugt ist, bildet die Blutmenge am inneren, weiteren Ende der konischen Kapillare eine überstehende Kuppe, die infolge der Schwerkraft etwas nach unten durchhängt. Je nach den speziellen Eigenschaften und der Temperatur des entnommenen Blutes kann die Kuppe aber auch, wie in Fig. 2 strichpunktiert dargestellt ist, ein größeres Volumen besitzen (siehe 17'). Da durch diese verschiedene Größe der überstehenden Kuppe Dosierfehler entstehen können, wird erfindungsgemäß der bereits in Fig. 1 dargestellte Abschlußstift 8 eingesetzt. Dieser Stift besitzt, wie aus den Fig. 3 bis 5 hervorgeht, vorzugsweise einen pyramidenförmigen Zentriervorsprung 9, der sich mit seinen Kanten auf dem Innenrand der oberen Öffnung der Kapillare 1 abstützt. Dabei werden aber dort, wo die Flächen der Pyramide durch die Ebene der Oberkante der Kapillare 1 hindurchgehen, schmale, etwa sicherförmige Luftaustrittsöffnungen 10 freigelassen, durch die beim Eintritt des durch Kapillarwirkung angesaugten Blutes die verdrängte Luft entweichen kann. In Fig. 3 und in Fig. 5, die einen Schnitt längs der Linie V-V in Fig. 3 darstellt, sind diese Luftaustrittsöffnungen 10 erkennbar.

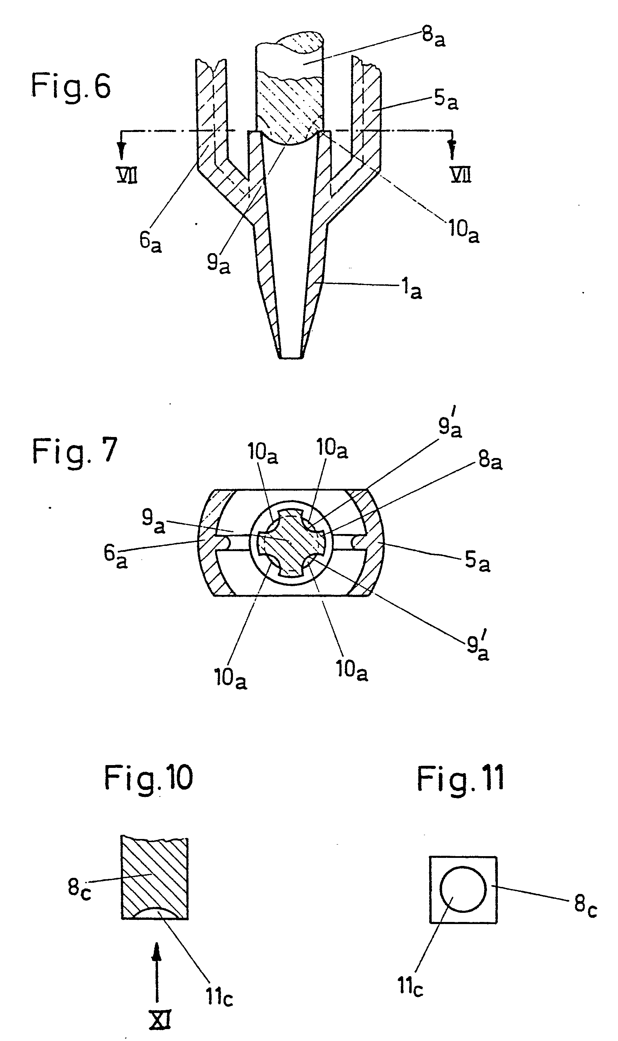

Der Zentriervorsprung kann auch anders geformt sein, beispielsweise kugelkalottenartig, wie in den Fig. 6 und 7 dargestellt. Dort sind im übrigen die gleichen Bezugszeichen wie in den Fig. 1 bis 5 verwendet, jedoch unter Hinzufügung eines Index »a«.

Hier sind, um Luftaustrittsöffnungen 10a zu schaffen, vier über den Umfang verteilte Einziehungen 9a' in der Kalotte vorgesehen.

Schließlich kann man auf einen Zentriervorsprung auch ganz verzichten und einen Abschlußstift 8b mit ebener Stirnfläche verwenden, wie er in den Fig. 8 und 9 dargestellt ist. Dort sind wieder die gleichen Bezugszeichen, jedoch unter Hinzufügung eines Index »b«, verwendet. Die Größe der quadratischen Stirnfläche des Abschlußstiftes 8b ist so gehalten, daß dieser Abschlußstift nur mit seinen vier Ecken auf den oberen Rändern der Kapillare 1b aufliegt, zwischen diesen aber Luftaustrittsöffnungen 10b freiläßt.

In gewissen Fällen ist es vorteilhaft, in der Stirnfläche des Abschlußstiftes eine Ausnehmung vorzusehen, die dann beim Ansaugen des Blutes die überstehende Kuppe, wie sie sich ohne Abschlußstift herausbildet, aufnehmen kann. Eine solche Ausbildung des unteren Endes des Abschlußstiftes ist in den Fig. 10 und 11 dargestellt. Der Abschlußstift ist dort mit 8c bezeichnet und besitzt eine kugelkalottenförmige Ausnehmung 11c in seiner im übrigen ebenen Stirnfläche. Da auch dieser Stift in der gleichen Weise auf dem oberen Rand der Kapillare 1 aufliegt, wie der in den Fig. 8 und 9 dargestellte Abschlußstift 8b, bilden sich auch dort Luftaustrittsöffnungen, die das Füllen der Kapillare mit dem angesaugten Blut nicht behindern. Die Ausnehmung 11c wird hierbei ohne weiteres voll mit Blut angefüllt. Damit erfolgt wiederum eine exakte und konstante Dosierung.

Der Halter 4 bzw. sein oberes Rohr 7 können im Durchmesser so ausgebildet werden, daß man das Rohr 7 oberseitig in den Hohlraum eines der Vorrichtung zugeordneten Stopfens 12 klemmend einsetzen kann, wie es in Fig. 12 dargestellt ist. Dieser Stopfen kann dann der Verschlußstopfen eines Probenröhrchens 13 sein, das zur Aufnahme der Vorrichtung nach entnommener Blutprobe dient. Das Probenröhrchen 13 ist dann in seinem unteren Teil mit Wasser oder einer anderen Flüssigkeit gefüllt, die sich zur Verdünnung der Blutprobe eignet. Der Abschlußstift mit Haltestab wird selbstverständlich vor dem Einstecken des oberen Endes des Rohres 7 in den Hohlraum des Stopfens 12 herausgezogen und weggeworfen. Damit kann durch leichte Schwenkbewegungen die in der Kapillare 1 befindliche Blutmenge ohne Schwierigkeiten und ohne Verluste ausgespült und mit der umgebenden Verdünnungsflüssigkeit vermischt werden.

Um Dosierfehler auch dadurch zu vermeiden bzw. vernachlässigbar klein zu halten, wird das untere Ende der Kapillare 1 möglichst dünnwandig ausgebildet. Das geschieht zweckmäßig dadurch, daß man dieses untere Ende außen mit einem steileren Konus versieht, als die übrige Kapillare. Sollte dann durch zu tiefes Eintauchen des unteren Endes 2 der Kapillare 1 in den Blutstropfen an der Entnahmestelle dennoch eine Benetzung des äußeren Konus erfolgen, die zu Dosierfehlern führen könnte, kann man diese äußere Konusfläche ohne weiteres abwischen.

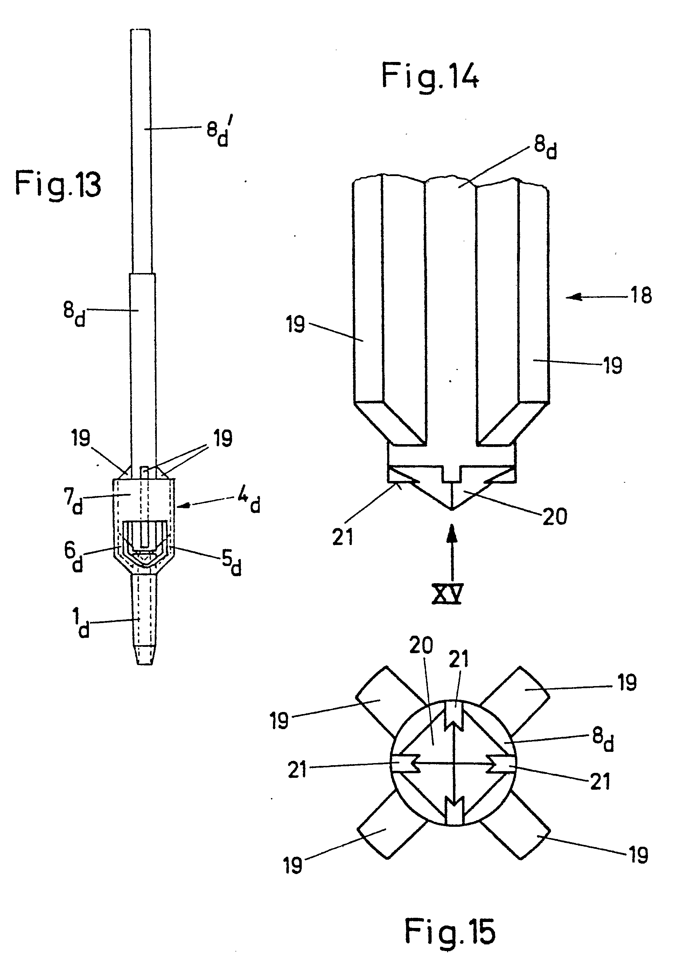

Bei der in den Fig. 13 bis 15 dargestellten Ausführung ist der Halter 4d für die Kapillare 1d nur relativ kurz gehalten; er besteht auch hier aus einem oberen, ringförmigen Teil 7d und zwei von diesem ausgehenden Stegen 5d, 6d, die die Kapillare 1d tragen.

Der Abschlußstift 8d ist an seinem hinteren, als Haltestab und Handgriff ausgebildeten Ende 8d' abgesetzt und besitzt an seinem vorderen Ende einen pyramidenförmigen Zentriervorsprung 20, der beim Einschieben des Halters schließlich mit seiner Spitze in die hintere Öffnung der Kapillare eintaucht. Dabei legen sich die von den Dachkanten der Pyramide vorstehenden vier Anschlagflächen 21, die besonders in den Fig. 14 und 15 erkennbar sind, auf die hintere Stirnfläche der Kapillare und halten damit den Zentriervorsprung 20 in einem vorbestimmten Abstand von dem hinteren Kapillarende.

Die vom unteren Ende 18 des Abschlußstiftes 8d radial nach außen vorstehenden Führungsflügel 19 passen stramm in den ringförmigen Oberteil 7d des Halters 4d, gestatten aber andererseits ein Einschieben und auch wieder Entfernen.

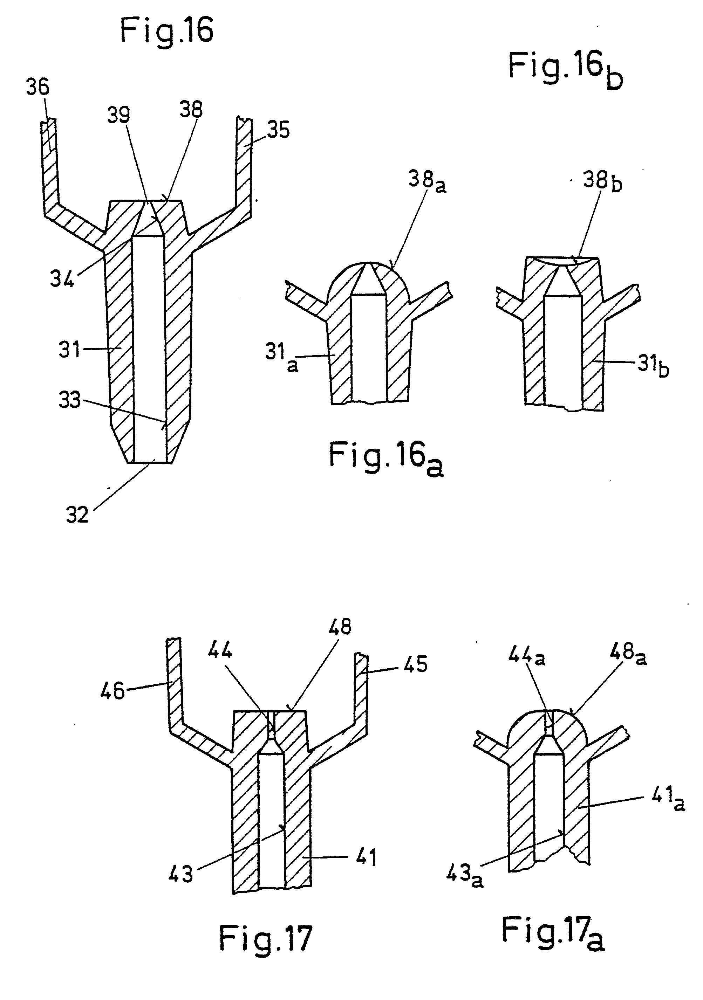

Bei den Ausführungsformen nach den Fig. 16 bis 18b ist anstelle eines Abschlußstiftes eine Querwand am hinteren Ende der Kapillare vorgesehen. Die in Fig. 16 dargestellte Kapillare 31, die mit den beiden Stegen 35 und 36 einstückig verbunden ist und aus transparentem Kunststoff hergestellt ist, besitzt eine leicht konische, fast zylindrische Bohrung 33, deren lichte Weite am vorderen Ende 32 nur so groß ist, daß dort das Blut mit Sicherheit noch durch Kapillarwirkung angesaugt wird.

Die Bohrung 33 verjüngt sich geringfügig in Richtung auf das hintere Ende, das sich bei 34 trichterförmig verengt und schließlich in eine sehr enge Luftaustrittsöffnung 39 übergeht, die sich in der Abschlußebene 38 der hinteren Querwand befindet.

Die in Fig. 16a dargestellte Kapillare 31a besitzt eine hintere Querwand, deren Abschlußfläche 38a halbkugelförmig ist, so daß die Querwand etwa konvex ausgebildet ist.

Die in Fig. 16b dargestellte Kapillare 31b besitzt dagegen eine leicht konkave hintere Querwand, deren Begrenzungsfläche 38b also ebenfalls konkav eingezogen ist.

Die in Fig. 17 dargestellte Kapillare 41 besitzt ebenfalls eine fast zylindrische Bohrung 43, die sich an ihrem hinteren Ende ebenfalls trichterförmig verengt, aber dort in einen feinen Kanal 44 übergeht, der in die ebene Abschlußfläche 48 der hinteren Querwand mündet und als Luftaustrittsöffnung dient.

Die Kapillare 41 wird von den beiden Stegen 45 und 46 des Halters getragen.

Die in Fig. 17a dargestellte Kapillare 41a besitzt, ähnlich wie die Ausführung nach Fig. 16a, eine abgerundete, etwa halbkugelförmige Abschlußfläche 48a. Die Bohrung 43a der Kapillare geht über eine trichterförmige Verengung in eine dünne, kanalförmige Luftaustrittsöffnung 44a über.

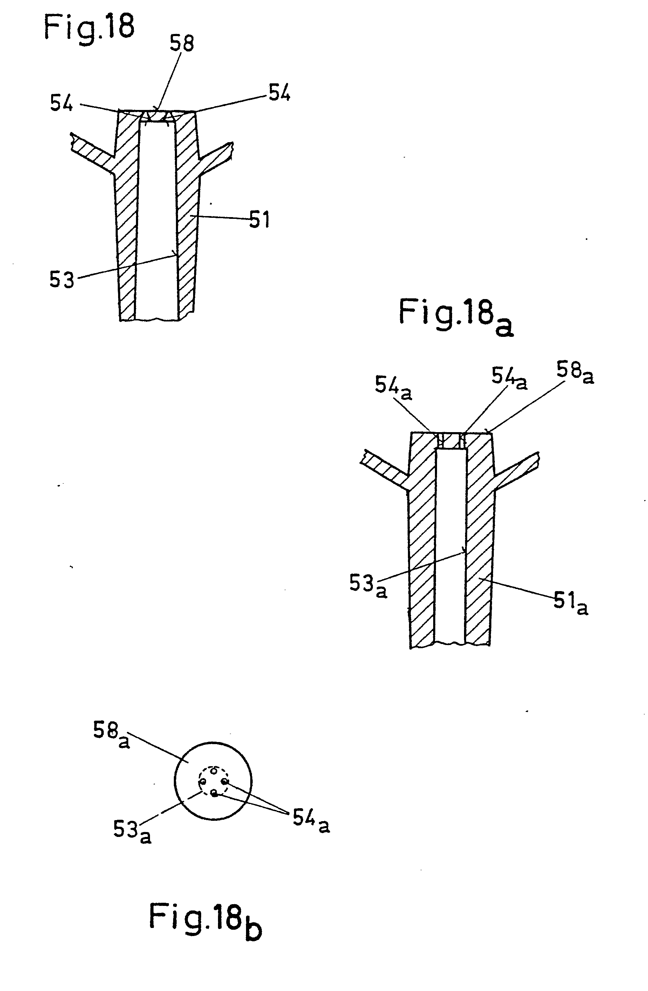

Fig. 18 zeigt eine Kapillare 51 mit einer stärker konischen Bohrung 53 und einer ebenen Querwand mit ebener Abschlußfläche 58, in die hier zwei trichterförmige Luftaustrittsöffnungen 54, ähnlich wie die Austrittsöffnung 39 in Fig. 16, münden.

Bei der abgewandelten Ausführungsform nach Fig. 18a und 18b sind wieder, ähnlich wie in Fig. 17, feine, kanalförmige Luftaustrittsöffnungen 54a vorgesehen, die in die Abschlußebene 58a münden. Auch hier ist die Bohrung 53a der Kapillare 51a von vorne nach hinten konisch verengt ausgebildet.

Wie man aus der Draufsicht auf die hintere Abschlußwand in Fig. 18b erkennt, sind insgesamt vier Luftaustrittsöffnungen 54a vorgesehen, die die vom eintretenden Blut verdrängte Luft aus der Bohrung 53a der Kapillare abführen.

## Patentansprüche

1. Blutaufnahmevorrichtung, bestehend aus einer beiderseits offenen Kapillare (1, 1a, 1b, 1d, 31, 31a, 31b, 41, 41a, 51, 51a) festgelegten Volumens, welche wenigstens am vorderen Ende derart eng ausgebildet ist, daß sie das aufzunehmende Blut durch Kapillarwirkung selbsttätig in ihren Innenraum einsaugt, sobald das vordere Ende mit der Blutentnahmestelle in Berührung gebracht wird und die nach hinten einstückig derart in einen Halter (4, 4d) übergeht, daß das vordere Ende der Kapillare über den Halter axial hinausragt, dadurch gekennzeichnet, daß ein Verschlußstift (8, 8a, 8b, 8c, 8d) in dem vom Mantel der Kapillare (1, 1a, 1b, 1d) ausgehenden, Spülfenster aufweisenden Halter (4, 4d) auf dem hinteren Ende der Kapillare (1, 1a, 1b, 1d) unter Freilassung kleiner Luftaustrittsöffnungen (10, 10a, 10b) abnehmbar angeordnet ist oder daß das hintere Ende der Kapillare (31, 31a, 31b, 41, 41a, 51, 51a) durch eine Querwand verschlossen ist, in der sich eine oder mehrere kleine Luftaustrittsöffnungen (39, 44, 44a, 54, 54a) befinden.

2. Vorrichtung nach Anspruch 1, dadurch gekennzeichnet, daß die Kapillare (1, 1a, 1b, 1d) sich nach hinten konisch erweitert, jedoch nur so weit, daß das eingesaugte Blut in waagerechter Lage der Kapillare nicht von selbst ausläuft.

3. Vorrichtung nach Anspruch 1 oder 2, dadurch gekennzeichnet, daß am hinteren Ende des Verschlußstiftes (8, 8d) einstückig ein axial verlaufender Führungs- und Haltestab (8', 8d') angeordnet ist.

4. Vorrichtung nach Anspruch 3, dadurch gekennzeichnet, daß der Haltestab (8', 8d') klemmend, aber axial verschiebbar im Halter (4, 4d) angeordnet ist.

5. Vorrichtung nach Anspruch 3 oder 4, dadurch gekennzeichnet, daß der Haltestab (8', 8d') für den Verschlußstift (8, 8a, 8b, 8d) als verlängerter Handgriff ausgebildet ist.

6. Vorrichtung nach einem der Ansprüche 3 bis 5, gekennzeichnet durch mindestens drei vom unteren Ende (18) des Haltestabes (8d) etwa radial auswärts vorstehende Führungsflügel (19), die das Ende (18) im Halter (4d) führen.

7. Vorrichtung nach einem der Ansprüche 1 bis 6, dadurch gekennzeichnet, daß beide Enden der Kapillare (1, 1a, 1d, 31, 31a, 31b, 41, 41a, 51, 51a) freistehen.

8. Vorrichtung nach einem der Ansprüche 1 bis 7, dadurch gekennzeichnet, daß sie aus Kunststoff, vorzugsweise einem transparenten Kunststoff, besteht.

9. Vorrichtung nach einem der Ansprüche 1 bis 8, dadurch gekennzeichnet, daß der Halter (4, 4d) zwei etwa parallel zueinander und zur Achse der Kapillare (1, 1a, 1d, 31, 41) verlaufende und zwischen sich Fenster freilassende Stege (5, 6; 5a, 6a; 5d, 6d; 35, 36; 45, 46) aufweist.

10. Vorrichtung nach Anspruch 9, dadurch gekennzeichnet, daß der Halter (4, 4d) an seinem der Kapillare abgewandten Ende in ein die beiden Stege miteinander verbindendes Rohr (7, 7d) übergeht.

11. Vorrichtung nach einem der Ansprüche 3 bis 10, dadurch gekennzeichnet, daß der Verschlußstift (8, 8a, 8d) an seinem freien Ende einen Zentriervorsprung (9, 9a, 20) mit Aussparungen am Umfang besitzt.

12. Vorrichtung nach Anspruch 11, dadurch gekennzeichnet, daß der Zentriervorsprung (9a) kalottenförmig ausgebildet ist.

13. Vorrichtung nach Anspruch 11, dadurch gekennzeichnet, daß der Zentriervorsprung (9, 20) pyramidenförmig ausgebildet ist.

14. Vorrichtung nach Anspruch 13, dadurch gekennzeichnet, daß von den Dachkanten des pyramidenförmigen Zentriervorsprunges (20) Anschlagnasen (21) mit radial verlaufenden Anschlagflächen vorstehen.

15. Vorrichtung nach einem der Ansprüche 3 bis 14, dadurch gekennzeichnet, daß der Verschlußstift (8c) an seinem freien Ende eine Ausnehmung (11c) in der Stirnfläche besitzt.

16. Vorrichtung nach einem der Ansprüche 3 bis 15, dadurch gekennzeichnet, daß der Außenmantel der Kapillare (1, 1a, 31) in der Nähe seines vorderen Endes (2) zu einem steileren Außenkonus angespitzt ist und die Kapillare am vorderen Ende eine extrem dünne Wandstärke besitzt.

17. Vorrichtung nach Anspruch 1, dadurch gekennzeichnet, daß die Querwand konvex ausgebildet ist, wobei sich eine Luftaustrittsöffnung (44a) in ihrer Mitte befindet.

18. Vorrichtung nach Anspruch 1, dadurch gekennzeichnet, daß die Querwand konkav ausgebildet ist, wobei sich eine oder mehrere Luftaustrittsöffnungen an ihrem Umfang befinden.

## Claims

1. Blood sampling device comprising a capillary (1, 1a, 1b, 1d, 31, 31a, 31b, 41, 41a, 51, 51a) open at both ends and of fixed volume, the capillary being so narrowly constructed, at least at its front end, that it automatically sucks the blood to the sampled into its interior by capillary action as soon as the front end is brought into contact with the blood extraction point, and merging at its rear end into a holder (4, 4d) in one piece in such a way that the front end of the capillary projects axially beyond the holder, characterised in that a closure pin (8, 8a, 8b, 8c, 8d) is removably arranged in the holder (4, 4d), which starts from the wall of the capillary (1, 1a, 1b, 1d) and has flushing windows, at the rear end of the capillary (1, 1a, 1b, 1d); or, in that the rear end of the capillary (31, 31a, 31b, 41, 41a, 51, 51a) is closed by a transverse wall in which one or more small air exit openings (39, 44, 44a, 54, 54a) is/are located.

2. Device in accordance with claim 1 and characterised in that the capillary (1, 1a, 1b, 1d) broadens conically in a rearward direction but only sufficiently far that the blood which is sucked in does not automatically run out in the horizontal position of the capillary.

3. Device in accordance with claim 1 or claim 2 and characterised in that an axially extending guide and holder rod (8', 8d') is integrally arranged at the rear end of the closure pin (8, 8d).

4. Device in accordance with claim 3 and characterised in that the holder rod (8', 8d') is clampingly but axially displaceably arranged in the holder (4, 4d).

5. Device in accordance with claim 3 or 4, characterised in that the holding rod (8', 8d') for the closure pin (8, 8a, 8b, 8d) is constructed as an elongated handle.

6. Device in accordance with one of the claims 3 to 5 characterised by at least three guide vanes (19) which project approximately radially outwardly from the lower end (18) of the holder rod (8d) and guide the end (18) in the holder (4d).

7. Device in accordance with one of the claims 1 to 6 and characterised in that the two ends of the capillary (1, 1a, 1d, 31, 31a, 31b, 41, 41a, 51, 51a) stand free.

8. Device in accordance with one of the claims 1 to 7 and characterised in that it consists of synthetic material, in particular a transparent synthetic material.

9. Device in accordance with one of the claims 1 to 8 and characterised in that the holder (4, 4d) has two webs (5, 6; 5a, 6a; 5d, 6d; 35, 36; 45, 46) which extend approximately parallel to one another and to the axis of the capillary (1, 1a, 1d, 31, 41) and which leave windows free between them.

10. Device in accordance with claim 9 and characterised in that the holder (4, 4d) merges at its end remote from the capillary into a tube (7, 7d) which joins the two webs together.

11. Device in accordance with one of the claims 3 to 10 and characterised in that the closure pin (8, 8a, 8d) has, at its free end, a centering projection (9, 9a, 20) with cut-outs at the periphery.

12. Device in accordance with claim 11 and characterised in that the centering projection (9a) is of spherical shape.

13. Device in accordance with claim 11 and characterised in that the centering projection (9, 20) is of pyramidal shape.

14. Device in accordance with claim 13 and characterised in that abutment noses (21) with radially extending abutment surfaces project from the roof edges of the pyramidal centering projection (20).

15. Device in accordance with one of the claims 3 to 14 and characterised in that the closure pin (8c) has a recess (11c) in the end face at its free end.

16. Device in accordance with one of the claims 3 to 15 and characterised in that the outer wall of the capillary (1, 1a, 31) is pointed in the vicinity of its front end (2) into a steeper outer cone and in that the capillary has an extremely thin wall at its front end.

17. Device in accordance with claim 1 and characterised in that the transverse wall is convexly shaped with an air outlet opening (44a) at its center.

18. Device in accordance with claim 1 and characterised in that the transverse wall is concavely shaped with one or more air outlet openings being located at its periphery.

## Revendications

1. Dispositif de prélèvement de sang constitué par un capillaire de volume prédéterminé ouvert des deux côtés (1, 1a, 1b, 1d, 31, 31a, 31b, 41, 41a,

51, 51a), qui est rendu suffisamment étroit, au moins à son extrémité antérieure, pour aspirer par capillarité le sang à prélever dès que l'extrémité antérieure est placée au contact du lieu de prélèvement du sang, et qui se prolonge vers l'arrière en un support (4, 4d) en une seule pièce de telle sorte que l'extrémité antérieure du capillaire est axialement en saillie sur le support, caractérisé en ce qu'une pointe d'obturation (8, 8a, 8b, 8c, 8d) est disposée de manière amovible dans le support (4, 4d) partant de l'enveloppe du capillaire (1, 1a, 1b, 1d) et présentant des fenêtres de rinçage, pour se placer sur l'extrémité postérieure du capillaire (1, 1a, 1b, 1d) en y ménageant de petites ouvertures de sortie d'air (10, 10a, 10b) ou en ce que l'extrémité postérieure du capillaire (31, 31a, 31b, 41, 41a, 51, 51a) est obturée par une paroi transversale présentant une ou plusieurs petites ouvertures de sortie d'air (39, 44, 44a, 54, 54a).

2. Dispositif selon la revendication 1, caractérisé en ce que le capillaire (1, 1a, 1b, 1d) s'élargit en cône vers l'arrière, mais dans une mesure limitée de telle sorte que le sang aspiré en position horizontale du capillaire ne s'en échappe pas de lui-même.

3. Dispositif selon la revendication 1 ou 2, caractérisé en ce qu'à l'extrémité arrière de la pointe d'obturation (8, 8d) est disposée d'une seule pièce une tige de guidage et de support (8', 8d') s'étendant axialement.

4. Dispositif selon la revendication 3, caractérisé en ce que la tige support (8', 8d') est ajustée mais susceptible de coulisser axialement dans le support (4, 4d).

5. Dispositif selon la revendication 3 ou 4, caractérisé en ce que la tige support (8', 8d') est formée en organe prolongé de préhension pour la pointe d'obturation (8, 8a, 8b, 8d).

6. Dispositif selon l'une des revendications 3 à 5, caractérisé par au moins trois ailettes de guidage (19) partant à peu près radialement de l'extrémité inférieure (18) de la tige support (8d), assurant le guidage de l'extrémité (18) dans le support (4d).

7. Dispositif selon l'une des revendications 1 à 6, caractérisé en ce que les deux extrémités du capillaire (1, 1a, 1d, 31, 31a, 31b, 41, 41a, 51, 51a) sont dégagées.

8. Dispositif selon l'une des revendications 1 à 7, caractérisé en ce qu'il est constitué en matière synthétique de préférence en une matière synthétique transparente.

9. Dispositif selon l'une des revendications 1 à 8, caractérisé en ce que le support (4, 4d) présente deux montants (5, 6; 5a, 6a; 5d, 6d; 35, 36; 45, 46) sensiblement parallèles entre eux et par rapport à l'axe du capillaire (1, 1a, 1d, 31, 41) et ménageant entre eux des fenêtres.

10. Dispositif selon la revendication 9, caractérisé en ce que le support (4, 4d) présente à son extrémité éloignée du capillaire une transition en un tube (7, 7d) reliant entre eux les deux montants.

11. Dispositif selon l'une des revendications 3 à 10, caractérisé en ce que la pointe d'obturation (8, 8a, 8d) présente à son extrémité libre une saillie de centrage (9, 9a, 20) avec des évidements à la périphérie.

12. Dispositif selon la revendication 11, caractérisé en ce que la saillie de centrage (9a) est en forme de calotte.

13. Dispositif selon la revendication 11, caractérisé en ce que la saillie de centrage (9, 20) est de forme pyramidale.

14. Dispositif selon la revendication 13, caractérisé par la disposition en saillie sur les arêtes de la saillie de centrage pyramidale (20) de faces d'appui (21) s'étendant radialement.

15. Dispositif selon l'une des revendications 3 à 14, caractérisé en ce que la pointe d'oburation (8c) présente à son extrémité libre un évidement (11c) dans la face frontale.

16. Dispositif selon l'une des revendications 3 à 15, caractérisé en ce que la paroi externe ou capillaire (1, 1a, 31) est effilée au voisinage de son extrémité antérieure (2) en un cône extérieur plus aïgu et le capillaire présente à son extrémité antérieure une épaisseur de paroi extrêmement fine.

17. Dispositif selon la revendication 1, caractérisé en ce que la paroi transversale présente une configuration convexe, une ouverture de sortie d'air (44a) étant ménagée en son milieu.

18. Dispositif selon la revendication 1, caractérisé en ce que la paroi transversale est de configuration concave, avec une ou plusieurs ouvertures de sortie d'air à sa périphérie.

Fig. 1

Fig. 2

**Fig.3**

**Fig.4**

**Fig.5**

Fig.6

8a

5a

VII

VII

6a

9a

10a

1a

Fig. 7

9'a

10a    10a

8a

9a

6a

5a

9'a

10a    10a

Fig.10

Fig.11

8c

8c

11c

XI

11c

## Fig. 12

12

7

6 5

1

13

## Fig. 8

8b

IX IX

1b

## Fig. 9

8b

10b 1b

Fig.14

Fig.13

# Fig.16

# Fig.16_b

# Fig.16_a

# Fig.17

# Fig.17_a

## Fig.18

## Fig.18a

## Fig.18b